# EUROPEAN PATENT SPECIFICATION

(11) **EP 4 460 310 B1**
(45) Date of publication and mention of the grant of the patent: **23.09.2026**
(21) Application number: 23737584.5
(22) Date of filing: 05.01.2023
(51) Int. Cl.: A61K 31/519, A61K 38/06, A61K 9/06, A61P 17/00, A61P 17/06

(54) **METHODS AND COMPOSITIONS FOR TREATMENT OF CUTANEOUS PROLIFERATIVE DISORDERS AND OTHER SKIN CONDITIONS**
VERFAHREN UND ZUSAMMENSETZUNGEN ZUR BEHANDLUNG VON HAUTPROLIFERATIVEN ERKRANKUNGEN UND ANDEREN HAUTERKRANKUNGEN
MÉTHODES ET COMPOSITIONS POUR LE TRAITEMENT DE TROUBLES CUTANÉS PROLIFÉRATIFS ET D'AUTRES AFFECTIONS CUTANÉES

(30) Priority: 05.01.2022 US 202263266433 P; 02.11.2022 US 202263382008 P
(43) Date of publication of application: 13.11.2024
(73) Proprietor: ASYMMETRIC THERAPEUTICS, LLC, Unadilla, New York 13849 (US)
(72) Inventor: FORD, John, P., New York 13849 (US)
(74) Representative: Prinz & Partner mbB
(86) International application number: PCT/US2023/010202
(87) International publication number: WO 2023/133199

(56) References cited:
- WO-A2-03/045334
- US-A1- 2014 248 270
- US-A1- 2019 105 261
- US-A1- 2019 365 798
- US-A1- 2020 009 040
- US-A1- 2020 276 108
- FEUERMAN E.J. ET AL.: "Allopurinol in psoriasis-a double-blind study", vol. 89, no. 1, 1 July 1973 (1973-07-01), pages 83 - 83, XP093314825, ISSN: 0007-0963, Retrieved from the Internet <URL:https://onlinelibrary.wiley.com/doi/pdf/10.1111/j.1365-2133.1973.tb01921.x> DOI: 10.1111/j.1365-2133.1973.tb01921.x

## Description

### FIELD OF THE INVENTION

The present embodiments are directed to the field of treatment of skin conditions. More particularly, the present embodiments pertain to compositions for the topical treatment of diseases that manifest increased cell proliferation, such as, for example, psoriasis and keratoacanthoma.

### BACKGROUND OF THE INVENTION

Many skin disorders with different causes manifest with increased proliferation rates of certain cells of the skin. Such skin disorders include psoriasis, keratoacanthoma, rosacea, and keloids. Cutaneous psoriasis, also known as plaque psoriasis, is a common ailment annually affecting more than three million patients in the United States alone. Cutaneous psoriasis is a chronic inflammatory disease that may manifest on the skin as a rash, dryness, fissures, flakiness, peeling, small bumps, thickness, and/or redness. Guttate psoriasis is another form of psoriasis that is less common but more acute than plaque psoriasis and manifests as small red spots that are typically teardrop-shaped.

Conventional topical treatments for cutaneous psoriasis include corticosteroids, vitamin D analogs, 0.05%-0.1% tazarotene, 3%-10% salicylic acid, methotrexate, and a 20% liquor carbonic distillate (LCD) solution. For mild to moderate psoriasis, topical vitamin D and steroids are often combined. Administration of vitamin D may inhibit cell replication, possibly through increasing cellular calcium uptake, but vitamin D also may increase the development of rosacea and is not effective for scalp involvement with psoriasis or in more aggressive cases of psoriasis. The most common form of psoriasis is plaque psoriasis, manifesting by a plaque of a markedly thickened stratum corneum or keratin crust. The plaque serves as a barrier to drug penetration. Methotrexate unfortunately suffers from having a narrow therapeutic window and is toxic with excess application. At present there is no satisfactory therapy for cutaneous psoriasis.

There have been many efforts to treat these conditions by exploiting immune inhibition. Anti-cytokine biologics targeting interleukin-17 (IL17) or its ligand (IL17A), interleukin-23 (IL23), or tumor necrosis factor alpha (TNF-α), or the small molecule drug apremilast (marketed under the trade name Otezla^{®} by Amgen Inc., Thousand Oaks, CA), a selective inhibitor of the enzyme phosphodiesterase 4 (PDE4) that inhibits production of TNF-α by rheumatoid synovial cells, among others, are increasingly being used to treat psoriasis. Although initially active, these agents at low incidence carry the risk of serious toxicities, including cancer and activating tuberculosis. Moreover, many of these therapies attempt merely to interrupt one step in a highly complex and redundant replication stimulatory network.

The inflammatory aspect of conditions such as psoriasis has also been a target of intense research for several decades. In 2005, Namazi ("Cannabinoids, loratadine and allopurinol as novel additions to the antisporiatic ammunition", Journal of the European Academy of Dermatology and Venerology, Vol. 19, pp. 319-322, (2005), incorporated by reference herein) suggested cannabinoids, loratadine, and allopurinol as agents that might be useful in treating these conditions.

Allopurinol was cited by Namazi as being a xanthine oxidase inhibitor with free radical scavenging activities with the speculative capacity to modify the inflammation associated with psoriasis. If allopurinol were to effectively treat psoriasis by disrupting xanthine oxidase-mediated free radical-scavenging activity, it would be expected that oxypurinol, allopurinol's exclusive active metabolite responsible for xanthine oxidase inhibition, would be even more effective to treat psoriasis by acting through the mechanism of xanthine oxidase inhibition. Allopurinol inhibits production of intercellular adhesion molecule-1 (ICAM-1), P2X, and tumor necrosis factor-alpha (TNF-α).

Allopurinol has been used topically by Rodemer in the prevention of a cutaneous toxicity of chemotherapeutic agents named "hand-foot" syndrome (see, for example, U.S. Patent No. 8,623,878). A subsequent clinical trial by Rodemer with allopurinol, however, completed accrual but did not meet its clinical efficacy endpoint.

Salim suggested the topical use of allopurinol or oxypurinol together with sulfonyl methane sulfonate for a large number of illnesses (see, for example, WO 94/05291 and WO 94/05293), but Salim's related published work was substantially retracted by the publisher as being fraudulent (see Hammerschmidt et al., "Allegations of Impropriety in Manuscripts by Aws S. Salim: Examination and Withdrawal of Journal Ægis", J. Lab. Clin. Med., Vol. 123, pp. 795-799 (1994)). Salim did not suggest psoriasis among a list of conditions to be treated by either agent. With respect to oral uses, allopurinol has been in long term clinical use as an oral treatment for gout and some types of kidney stones.

There are puzzling and contradictory reports on the oral use of allopurinol for the treatment of psoriasis. Viglioglia et al. ("Allopurinol in Psoriasis", Dermatologica, Vol. 141, pp. 203-207 (1970)) reported excellent results in 50% of cases in treating psoriasis with allopurinol, good results in 34% of cases, and moderate results in 16% of cases.

In a subsequent double-blind cross-over study, however, the oral administration of allopurinol showed no improvement over a placebo in the treatment of psoriasis (see Feuerman et al., "Allopurinol in psoriasis-a double blind study", British Journal of Dermatology, Vol. 89, pp. 83-86 (1973)).

As noted above, the active species in the inhibition of xanthine oxidase by allopurinol is oxypurinol. Oxypurinol is the only known metabolite of allopurinol responsible for xanthine oxidase inhibition. If Namazi's suggestion of xanthine oxidase inhibition were correct, it would be expected that oxypurinol would be a superior treatment for psoriasis compared to allopurinol, and allopurinol would not be used as a therapeutic.

An additional reason for studying oxypurinol rather than allopurinol is that allopurinol has been long known, unlike oxypurinol, to cause in people deleterious depletion of a vital energy intermediate phosphoribosyl pyrophosphate (PRPP) (see, for example, Fox et al., "Depletion of Erythrocyte Phosphoribosylpyrophosphate in Man: A Newly Observed Effect of Allopurinol", The New England Journal of Medicine, Vol. 283, pp. 1177-1182 (1970), incorporated by reference herein). Thus, if true, oxypurinol rather than allopurinol would be both a more active and safer drug than allopurinol. After the expenditure of significant time and resources and completely unexpectedly, the present studies showed that allopurinol is both much more active and less toxic than oxypurinol.

In fact, as the above careful review of the published literature of oxypurinol and allopurinol made a very compelling case for the superiority of oxypurinol over allopurinol as a topical agent, significant resources in time and money were expended to prepare, submit, and defend U.S. Patent Application Publication No. 2020/0046703, published February 13, 2020, for the use of oxypurinol over allopurinol. This patent application was abandoned after confirmatory laboratory tests of oxypurinol superiority over allopurinol failed decisively.

There is a compelling need for safe and effective treatments for psoriasis and other skin disorders marked by increased cellular proliferation. Broadly inhibiting cell proliferation carries with it risks of significant side effects. As an example, inhibiting all growth after tissue injury disrupts the natural healing process and is a concern for any cell growth-inhibiting strategy.

An ideal therapeutic would decrease the cell replication by inhibiting a nonredundant pathway vital for cell replication while at the same time not inhibiting vital cellular baseline and salvage function to preserve viability.

The present studies initially sought to exploit the possibility of treating psoriasis by xanthine oxidase inhibition by using the allopurinol metabolite oxypurinol that is the obligate allopurinol metabolite that inhibits xanthine oxidase.

### SUMMARY OF THE INVENTION

The object of the invention is solved by a topical pharmaceutical formulation according to claim 1.

In some aspects of the disclosure, topical pharmaceutical formulations comprising allopurinol or a pharmaceutically acceptable salt thereof and a co-agent or a pharmaceutically acceptable salt thereof are provided, wherein the co-agent is glutathione, the topical pharmaceutical formulation further comprising a carrier.

### BRIEF DESCRIPTION OF THE DRAWINGS

FIG. 1 shows the number of total HaCaT cells relative to the initial number of live cells in the presence of certain amounts of allopurinol, oxypurinol, and methotrexate in an M5 psoriasis cocktail.
FIG. 2 shows the number of dead HaCaT cells relative to the initial number of live cells in the presence of certain amounts of allopurinol, oxypurinol, and methotrexate in an M5 psoriasis cocktail.
FIG. 3 shows an image of a MatTek human psoriatic tissue culture system (MatTek Corporation, Ashland, MA) after 5 days of exposure to a 3 mM allopurinol treatment medium.
FIG. 4 shows an image of a MatTek human psoriatic tissue culture system after 5 days of exposure to a 3 mM allopurinol and 3 mM glutathione treatment medium.
FIG. 5A shows a first image of a MatTek human psoriatic tissue culture system after 5 days of exposure to a 100 nM Vitamin D treatment medium.
FIG. 5B shows a second image of a MatTek human psoriatic tissue culture system after 5 days of exposure to a 100 nM Vitamin D treatment medium.
FIG. 6 shows the dermis thickness in a MatTek human psoriatic tissue culture system treated with allopurinol and a combination of allopurinol and glutathione versus a control.
FIG. 7 shows a bar graph of relative Ki67 levels in the MatTek human psoriatic tissue culture system on Day 6.
FIG. 8 shows relative biomarker levels of Ki67, P21, IL17A, IL23A, TNFα, and TGFβ after MatTek human psoriatic tissue cultures were exposed to allopurinol, glutathione, a combination of allopurinol and glutathione and controls.
FIG. 9 shows the ratio of P21 to Ki67 mRNA levels in a MatTek human psoriatic tissue culture system.
FIG. 10A shows a psoriasis plaque on the left shoulder prior to treatment.
FIG. 10B shows the left shoulder of FIG. 10A after one month of treatment.
FIG. 11A shows a Dupuytren's contracture of the right fifth finger prior to treatment.
FIG. 11B shows the right fifth finger of FIG. 11A after one month of treatment.
FIG. 11C shows the right fifth finger of FIG. 11A after two months of treatment.
FIG. 11D shows the right fifth finger of FIG. 11A after four months of treatment.
FIG. 12A shows a long-standing allergic reaction on the calves prior to treatment.
FIG. 12B shows the backs of the calves of FIG. 12A after five days of treatment.
FIG. 13A shows three areas of guttate psoriasis prior to treatment.
FIG. 13B shows the three areas of FIG. 13A after a day and a half of treatment.
FIG. 13C shows the three areas of FIG. 13A after two days of treatment.
FIG. 13D shows the three areas of FIG. 13A after two and a half days of treatment.
FIG. 13E shows the three areas of FIG. 13A after six and a half days of treatment.
FIG. 14A shows an allergic reaction on the shins prior to treatment.
FIG. 14B shows the shins of FIG. 14A after seven days of treatment.

### DETAILED DESCRIPTION

It was surprisingly found in tissue culture that not only was allopurinol more effective than oxypurinol in decreasing psoriasis-induced abnormal cell growth, but allopurinol also decreased cell death more effectively than oxypurinol.

Keratinizing cells have slow growth but not no growth (see, for example, Squier et al., ed., Human Oral Mucosa: Development, Structure, and Function, Wiley-Blackwell, p. 29 (2011)). Thus, preservation of tissue integrity is vital. Again and surprisingly, it was found that allopurinol causes less cell death than oxypurinol in a tissue culture model. At the highest human dose concentration tested, topical allopurinol was effective and well tolerated.

In exemplary embodiments, a co-agent is administered with the allopurinol. In many embodiments, the co-agent is an antioxidant. In some embodiments, the co-agent is a skin permeation enhancer. In some embodiments, the co-agent is a sulfur-bearing antioxidant. In many embodiments, the co-agent includes a thiol or prothiol. Appropriate thiols may include, but are not limited to, glutathione, N-acetyl cysteine, cysteine, theanine, cystine, β-mercaptoethanol, and/or dithiothreitol. In particular embodiments, the co-agent is glutathione.

The formulations of the disclosure have been shown to be effective against psoriasis. The plaque of psoriasis serves as a barrier to topical drug penetration. While it was expected that an agent like glutathione might be useful to improve allopurinol delivery, it was surprisingly found, however, that co-administered glutathione was not merely capable of improving drug delivery, but also reduced swelling in psoriasis not observed with allopurinol alone. Example 2 shows that compared with the treatment with allopurinol or glutathione individually, the coadministration of topical allopurinol and glutathione is synergistic in tissue culture models of psoriasis where there is no plaque. Glutathione can significantly reduce the thickness of the vital dermis layer to help control psoriasis.

In view of the contradictory and confusing prior art reports on oral administration of allopurinol in the treatment of psoriasis, allopurinol and the allopurinol metabolite and direct inhibitor of xanthine oxidase, oxypurinol, were tested for topical application. The issue of reduced GI uptake of oxypurinol relative to allopurinol is not a factor for topical application like it is with oral use.

It was expected that the phosphoribosyl diphosphate (PRPP) depletion caused by allopurinol but not oxypurinol, as noted clinically in 1970, would cause cell toxicity, as implied by Fox et al. ("Depletion of Erythrocyte Phosphoribosylpyrophosphate in Man: A Newly Observed Effect of Allopurinol", The New England Journal of Medicine, Vol. 283, pp. 1177-1182 (1970)), which would negatively impact allopurinol's effectiveness relative to oxypurinol.

Surprisingly, the allopurinol control was dramatically more effective than the oxypurinol in an in vitro model of human psoriasis in reducing cell proliferation without increasing cell death. Without being bound by theory, this may be due to, rather than in spite of, the PRPP depletion caused by allopurinol.

Methotrexate was taken as a model treatment composition that inhibits cell replication, and the inhibition of cell replication has been correlated to response to methotrexate in the treatment of psoriasis. Methotrexate unfortunately suffers from having a narrow therapeutic window and is toxic with excess application.

Provided herein are methods and compositions for the treatment of skin disorders, such as allergic cutaneous reactions or cutaneous cellular proliferative disorders or conditions, such as, for example, proliferative skin disorders or conditions, such as, for example, cutaneous psoriasis, keratoacanthoma, rosacea, keloids, hand-foot syndrome, and cancer. In addition, disorders such as Dupuytren's Contracture and allergic may be effectively treated by compositions of the present disclosure.

Embodiments of the present disclosure, in comparison to concepts failing to include one or more of the features disclosed herein, reduce the proliferation rate of cells associated with a cutaneous cellular proliferative disorder, preferentially reduce the proliferation rate of cells of a cell type with a higher proliferation rate at a treatment site including other cell of a cell type having a lower proliferation rate, reduce or prevent one or more symptoms associated with a cutaneous cellular proliferative disorder, reduce the likelihood or severity of one or more symptoms associated with a cutaneous cellular proliferative disorder, reduce the frequency of recurrence of one or more symptoms associated with a cutaneous cellular proliferative disorder, or combinations thereof.

In exemplary embodiments, a protective formulation limits cell proliferation without increasing cell death of target skin cells. Methotrexate has been used in the treatment of certain skin disorders, but methotrexate limits cell proliferation by limiting the number of live cells by increasing cell death of skin cells. As disclosed herein, however, allopurinol was observed in vitro to limit skin cell proliferation with a minimal increase in cell death much more effectively in a psoriasis-inducing environment than methotrexate.

In exemplary embodiments, a protective formulation includes a therapeutic amount of allopurinol or a pharmaceutically acceptable salt thereof. In exemplary embodiments, a protective formulation is topically applied at the site of a skin condition. In some embodiments, the skin condition is a cutaneous cellular proliferative disorder. In exemplary embodiments, the therapeutic amount of allopurinol slows the proliferation of cells associated with a cutaneous cellular proliferative disorder. In some embodiments, the protective formulation treats at least one symptom associated with a cutaneous cellular proliferative disorder. In some embodiments, the skin condition is an allergic cutaneous reaction. In some embodiments, the protective formulation is a topical formulation.

In some embodiments, the protective formulation also includes a therapeutic amount of a co-agent. In some embodiments, the co-agent is an antioxidant. In some embodiments, the co-agent is a skin permeation enhancer. In some embodiments, the co-agent is a sulfur-bearing antioxidant. In many embodiments, the co-agent includes a thiol or prothiol. Appropriate thiols may include, but are not limited to, glutathione, N-acetyl cysteine, cysteine, theanine, cystine, β-mercaptoethanol, and/or dithiothreitol. In some embodiments, the co-agent provides a synergistic effect when used in amounts such as predetermined amounts, in combination with the allopurinol. In some embodiments, the co-agent aids in delivery of the allopurinol to the treatment site. In some embodiments, the co-agent disrupts keratin disulfide crosslinks in a skin plaque.

In some embodiments, the co-agent is glutathione or a pharmaceutically acceptable salt thereof. In some embodiments, the glutathione softens the skin, improves oxidative stress in the stratum corneum, dissolves keratin multimers, reduces swelling, and/or aids in the penetration of the topical formulation into the skin. In some embodiments, the glutathione increases permeation of the protective formulation into a psoriatic plaque.

In some embodiments, the protective formulation further includes one or more agents that aid in stabilizing and/or preserving the glutathione.

In some embodiments, the protective formulation includes a therapeutic amount of glutathione or a pharmaceutically acceptable salt thereof, optionally with a second active ingredient.

Embodiments of the present disclosure further include pharmaceutical compositions including allopurinol and glutathione, and optionally further including one or more pharmaceutically acceptable excipients. Such pharmaceutical compositions may be administered orally or configured to be delivered as any effective conventional dosage forms, including, for example, immediate, slow and timed-release oral preparations, parenterally, topically, nasally, ophthalmically, optically, sublingually, rectally, vaginally, and the like. The disclosure further includes methods of treating disease including administering to patients in need thereof allopurinol and glutathione such as, for example, pharmaceutical compositions comprising allopurinol, glutathione, and one or more pharmaceutically acceptable excipients.

As discussed in more detail in the Examples section, allopurinol was observed in vitro to reduce both cell proliferation and cell death in a model human psoriasis system relative to methotrexate. Topical treatments of allopurinol and glutathione were observed in the Examples to treat a human psoriatic plaque, human guttate psoriasis, Dupuytren's contracture, and an allergic cutaneous reaction. With respect to the human psoriatic plaque, the treatment was curative. In all other cases, improvements were observed.

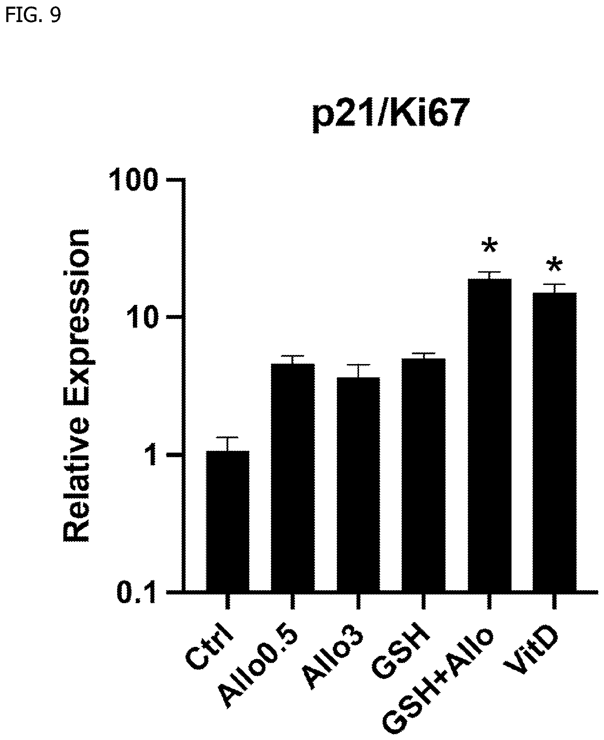

Ki67 is a replication marker for cells and the lower the value, the lower the replication rate. P21 is a protein which is a marker for the inhibition of cell proliferation. Thus, an increased ratio of P21/Ki67 indicates decreased cell replication. FIG. 9 illustrates this ratio under several conditions as described in Example 2.

The increase of allopurinol exposure from an increase in concentration of allopurinol in a topical application from 0.5 mM to 3.0 mM did not increase the p21/Ki67 ratio and the effect did not achieve statistical significance. However, the application of allopurinol and glutathione together did increase the p21/ Ki67 ratio and did achieve statistical significance. Thus the effects of the combined application of glutathione and allopurinol synergistically limited cell replication.

A combined treatment of allopurinol and glutathione also caused a decrease in a psoriatic skin model dermis layer tissue thickness at 6 days compared to dermis thickness at 2 days of treatment. A decrease in the dermis layer thickness has been previously used to establish successful treatment response in human psoriatic patients (see, for example, Phillips et al., "Dermal Reflectivity Determined by Optical Coherence Tomography is an Indicator of Epidermal Hyperplasia and Dermal Edema within Inflamed Skin", Journal of Biomedical Optics, Vol. 16, Art. 040503 (2011)). A 3-mM concentration of allopurinol treatment alone caused an increase in dermis layer over this time period (see Example 2).

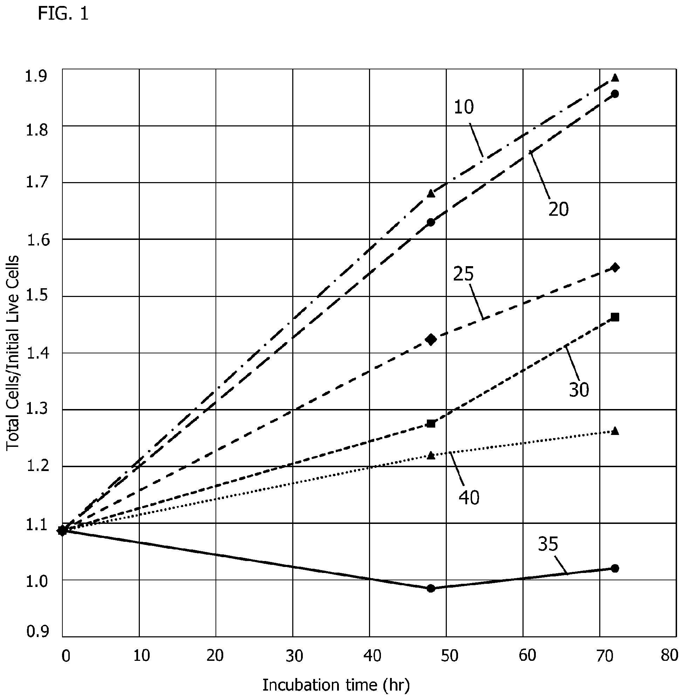

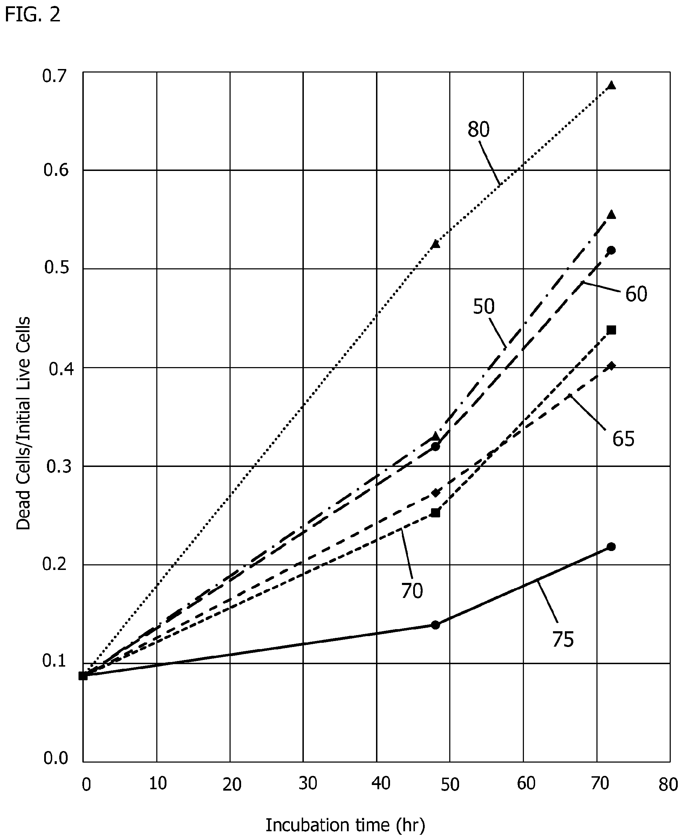

Allopurinol, when compared with standard care methotrexate, better inhibited cell replication in an HaCaT human cell monolayer model of psoriasis and also better preserved cell survival as seen in Example 1, FIG. 1, and FIG. 2.

The application of glutathione alone was shown to inhibit Ki67.

On this basis, it is expected that allopurinol, both alone and in combination with glutathione, is of benefit in a topical protective treatment against all agents that cause a toxicity to the hands and feet but not to the relatively still slower-growing non-palmar and non-plantar human skin, as well as any proliferative disorders or conditions that would be subject to treatment by a reduction in the proliferation of cells at the affected site of the disorder or condition.

In summary, the application of allopurinol showed an unexpected inhibition of cell replication with preservation of cell survival in a HaCaT human cell monolayer in vitro model of psoriasis that was significantly improved compared with a standard agent, methotrexate, in the treatment of psoriasis. Not wishing to be bound by theory, the inhibition of growth may reflect consumption of PRPP by hypoxanthine xanthine phosphoribiosyl transferase as initially described by Nelson et al. ("Formation of nucleotides of (6-14C)allopurinol and (6-14C)oxipurinol in rat tissues and effects on uridine nucleotide pools", Biochem. Pharmacol., Vol. 22, pp. 2003-2022, (1973)). Unexpected in the MatTek tissue model of psoriasis, the application of glutathione alone resulted in the strongest inhibition of the expression of the cell replication marker Ki67 compared with allopurinol and also comparable to exposure to Vitamin D, a standard, but insufficient, psoriasis therapeutic. Glutathione also induced expression of the replication inhibitor p21, as well as inhibiting the expression of the inflammatory markers IL17A, IL23, TNF alpha, and TNF beta.

Allopurinol and glutathione combined treatment caused a decrease in PSE dermis layer tissue thickness at 6 days compared to dermis thickness at 2 days treatment. Allopurinol treatment alone, at 3.0 mM, paradoxically caused an increase in dermis layer over this time period. This result was consistent with a synergistic effect of an improvement in tissue homeostasis with the addition of glutathione to allopurinol. A decrease in the dermis layer thickness has been previously used to monitor successful treatment response in human psoriatic patients. A higher p21/KI67 ratio was observed with glutathione and allopurinol treatment together compared with allopurinol or glutathione treatment alone. Again the increase in allopurinol exposure from 0.5 mM to 3.0 mM, like the result with dermis thickness, provided no evidence of response benefit for increased allopurinol exposure, a result again consistent with glutathione and allopurinol synergy.

Both allopurinol and glutathione have individually and separately been used topically and safely in human. Allopurinol has been administered widely and safely orally to humans for the treatment of gout for more than 50 years. Glutathione is an antioxidant and is a normal cell constituent present intracellularly at concentrations in 1-10 mM range. Use of allopurinol and glutathione at 3% topically twice a day for 7 weeks resulted in the disappearance of a 3 cm area of psoriatic plaque present for 2 years.

In some embodiments, the allopurinol is in the form of a pharmaceutically-acceptable salt that may provide one or more advantageous properties over allopurinol alone.

In some embodiments, the glutathione is in the form of a pharmaceutically-acceptable salt that may provide one or more advantageous properties over glutathione alone.

The topical efficacy and superiority of allopurinol over oxypurinol is surprising and unexpected. Applicant is unaware of any reference or rationale in the literature that would suggest the superiority of a topical efficacy for allopurinol over oxypurinol.

Although allopurinol has not previously been tested for topical treatment of psoriasis, allopurinol has generally been observed in other treatments to cause only about a 2% to 3% incidence of rash when applied topically. It is nearly unheard of in the literature for any agent applied to intact non-mucosal skin to have a serious skin reaction (see, for example, Sachs et al., "Anaphylaxis and toxic epidermal necrolysis or Stevens-Johnson syndrome after nonmucosal topical drug application: fact or fiction?", Allergy, Vol. 62, pp. 877-883 (2007)), indicating that a topical composition carries little risk of serious systemic complications.

In some embodiments, compositions and methods include allopurinol as an active ingredient in a protective formulation. In some embodiments, compositions and methods include allopurinol as an active ingredient in combination with the co-agent glutathione in a protective formulation.

In some embodiments, the protective formulation is formulated for topical application to skin, and the protective formulation can usefully be formulated as a topical formulation. Appropriate topical formulations may include, but are not limited to, an ointment, a cream, a lotion, a paste, an aerosol spray, a roll-on liquid, stick, or pad, or an aerosol foam (mousse) composition. In some embodiments, compositions and methods include topical delivery of a protective formulation including allopurinol as an active ingredient as described in U.S. Patent No. 9,084,788, entitled "Compositions and methods for treating and preventing dermatoses" and issued to Ford on July 21, 2015, which discloses compositions and methods for topical administration.

In some embodiments, the treatment includes topical application of the protective formulation to the skin at least once a day, such as for example twice a day. In some embodiments, the treatment includes continuing the topical application for at least two days, such as, for example, three days, four days, five days, six days, one week, two weeks, three weeks, one month, two month, three months, or longer than three months.

The preferred clinical composition of the protective formulation may depend upon the tissue desired to be protected. Pharmaceutical formulation is a well-established art in that it is known that therapeutics must be formulated and there are a myriad of variables involved in such formulation (see, for example, Allen ed., Remington: The Science and Practice of Pharmacy, 22nd ed., Pharmaceutical Press (2012); Allen, Ansel's Pharmaceutical Dosage Forms and Drug Delivery Systems, 11th ed., Wolters Kluwer (2017); and Rowe et al., ed., Handbook of Pharmaceutical Excipients, 6th ed., Pharmaceutical Press (2009)). Therefore, challenges often remain for preparing clinical and commercial formulations and formulations with particular functionalities.

Appropriate topical formulations may, for example, be anhydrous, aqueous, or water-in-oil or oil-in-water emulsions. Appropriate topical formulations may further include one or more pharmaceutically acceptable carriers and various skin actives. Amounts of the carrier may range from about 1 to about 99%, preferably from about 5 to about 70%, optimally from about 10 to about 40% by weight. Among useful carriers are emollients, water, inorganic powders, foaming agents, emulsifiers, fatty alcohols, fatty acids, or combinations thereof.

Emollients may be selected from polyols, esters, and hydrocarbons. Polyols suitable for the appropriate topical formulations may include propylene glycol, dipropylene glycol, polypropylene glycol, polyethylene glycol, sorbitol, hydroxypropyl sorbitol, hexylene glycol, 1,3-butylene glycol, 1,2,6-hexanetriol, glycerin, ethoxylated glycerin, propoxylated glycerin, xylitol, or mixtures thereof.

Esters useful as emollients include alkyl esters of fatty acids having 10 to 20 carbon atoms. Methyl, isopropyl, and butyl esters of fatty acids may be useful. Examples include hexyl laurate, isohexyl laurate, isohexyl palmitate, isopropyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate. Particularly preferred are C12-C15 alcohol benzoate esters.

Esters useful as emollients may also include alkenyl esters of fatty acids having 10 to 20 carbon atoms, such as, for example, oleyl myristate, oleyl stearate and oleyl oleate. Esters useful as emollients may also include ether-esters such as fatty acids esters of ethoxylated fatty alcohols.

Esters useful as emollients may also include polyhydric alcohol esters, such as, for example, ethylene glycol mono- and di-fatty acid esters, diethylene glycol mono- and di-fatty acid esters, polyethylene glycol (200-6000) mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and/or polyoxyethylene sorbitan fatty acid esters.

Esters useful as emollients may additionally include wax esters, such as, for example, beeswax, spermaceti, myristyl myristate, and/or stearyl stearate, and sterol esters, such as, for example, cholesterol fatty acid esters.

Appropriate hydrocarbon carriers may include mineral oil, polyalphaolefins, petrolatum, isoparaffin, polybutenes, and/or mixtures thereof.

Inorganic powders may also be useful as carriers in topical formulations. Examples may include clays (such as, for example, Montmorillonite, Hectorite, Laponite and Bentonite), talc, mica, silica, alumina, zeolites, sodium sulfate, sodium bicarbonate, sodium carbonate, calcium sulfate, and/or mixtures thereof.

Appropriate topical formulations may also include aerosol propellants, serving as, or in addition to, carriers. Propellants may be based on volatile hydrocarbons such as propane, butane, isobutene, pentane, isopropane, and mixtures thereof. Phillips Petroleum Company (Bartlesville, OK) may be a source of such propellants under trademarks including A3, A32, A51, and/or A70. Halocarbons including fluorocarbons may further widely be employed as propellants.

Appropriate topical formulations for administration to the skin may include emulsifiers, either serving as, or in addition to, carriers.

Appropriate emulsifiers may be selected from nonionic, anionic, cationic, and/or amphoteric emulsifying agents. Appropriate emulsifiers may range in amount anywhere from about 0.1 to about 20% by weight.

Appropriate nonionic emulsifiers may include alkoxylated compounds based on C10-C22 fatty alcohols and acids and sorbitan. Appropriate materials may be available, for instance, under the Neodol trademark (Shell Oil Company, Houston, TX), as copolymers of polyoxypropylenepolyoxyethylene sold under the Pluronic trademark (BASF Corporation, Ludwigshafen, Germany), and/or as alkyl polyglycosides available from the Henkel Corporation (Düsseldorf, Germany).

Appropriate anionic type emulsifiers may include fatty acid soaps, sodium lauryl sulfate, sodium lauryl ether sulfate, alkyl benzene sulfonate, mono- and dialkyl acid phosphates, sarcosinates, taurates, and/or sodium fatty acyl isethionate.

Appropriate amphoteric emulsifiers may further include dialkylamine oxide and various types of betaines, such as, for example, cocamidopropyl betaine.

Appropriate topical formulations may also include preservatives, such as, for example, methyl paraben and propyl paraben, which are useful to prevent microbial contamination.

In some embodiments, compositions and methods include oral delivery of a protective formulation including oxypurinol as an active ingredient as described in US 9 119 855 B2, entitled "Compositions and methods for treatment of the side-effects associated with administration of cancer chemotherapeutic agents" and issued to Ford on September 1, 2015, which discloses compositions and methods for oral administration.

In some embodiments, each active ingredient may be present in the protective formulation in a weight percentage of at least 0.01%, 0.05%, 1.0%, 1.5%, 2.0%, 2.5%, 3.5%, 4.0%, 4.5%, 5.0%, 10%, 15%, 20%, 25%, 30%, 35%, 40%, 45%, even 50% or more, with intermediate values permissible, and is typically present to a weight/weight percentage of no more than about 50%, 45%, 40% 30%, 35%, 30%, 25%, 20%, 15%, 10%, 5%, 45%, 4.0%, 3.5%, 3.0%, 2.5%, 2.0%, 1.5%, 1.0%, and even, at times, to a weight/weight percentage of no more than about 0.05%, even as little as 0.01%, with intermediate values permissible.

In some embodiments, the allopurinol is present in the composition in an amount, by weight, in the range of about 1% to about 5%, alternatively about 2% to about 4%, alternatively about 3%, or any value, range, or sub-range therebetween.

In some embodiments, the glutathione is present in the composition in an amount, by weight, in the range of about 0.1% to about 5%, alternatively about 1% to about 5%, alternatively about 2% to about 4%, alternatively about 3%, or any value, range, or sub-range therebetween.

In some embodiments, the protective formulation is applied or delivered locally to the site of protection to provide a sustained concentration of the active ingredient of at least 0.03 mM, alternatively 0.03 mM to 30 mM, alternatively 0.03 mM to 3 mM, alternatively 0.03 mM to 0.1 mM, alternatively at least 0.1 mM, alternatively 0.1 mM to 0.3 mM, alternatively at least 0.3 mM, alternatively 0.3 mM to 1 mM, alternatively at least 1 mM, alternatively 1 mM to 30 mM, alternatively 1 mM to 10 mM, alternatively 1 mM to 3 mM, alternatively at least 3 mM, alternatively 3 mM or less, alternatively 1 mM or less, alternatively 0.3 mM or less, alternatively 0.1 mM or less, or any value, range, or sub-range therebetween.

In some embodiments, the active ingredient includes allopurinol or a pharmaceutically acceptable salt thereof. In some embodiments, allopurinol is the primary active ingredient in the protective formulation. In some embodiments, allopurinol is the only active ingredient in the protective formulation. In some embodiments, the protective formulation is free or substantially free of other active ingredients. Substantially free, as used herein, refers to an amount of less than 0.001 mM of a compound in a composition.

In these and other embodiments, the protective formulation may also include a co-agent as an active ingredient. In some embodiments, the co-agent provides a synergistic effect in combination with the allopurinol. An example of such a co-agent is glutathione.

The negligible toxicity of locally therapeutic doses of allopurinol indicates a significant benefit to proactive administration topically and locally of a protective formulation including a therapeutic amount of allopurinol to the relevant tissue of a patient susceptible to a cutaneous cellular proliferative disorder. The therapeutic dose includes a therapeutic amount of the allopurinol sufficient to slow the proliferation of cells of the relevant tissue from development of the cutaneous cellular proliferative disorder.

In some embodiments, the protective formulation including a therapeutic amount of allopurinol is administered topically and locally to the relevant tissue of a patient to lessen the symptoms of and treat a cutaneous cellular proliferative disorder. The therapeutic dose includes a therapeutic amount of the allopurinol sufficient to slow the proliferation of cells of the relevant tissue and treat the cutaneous cellular proliferative disorder.

In some embodiments, a method of site-specifically reducing the replication rate of skin cells in a patient includes topically applying or locally delivering an effective amount of allopurinol to a site on the patient's skin where reduced skin cell replication is desired.

### EXAMPLES

The invention is further described in the context of the following examples which are presented by way of illustration, not of limitation.

### Example 1

Immortalized human keratinocytes (HaCaT cells) purchased from AddexBio (San Diego, CA) were maintained in vitro in Dulbecco's modified Eagle's medium (DMEM) supplemented with 10% Fetal Bovine Serum (FBS) and antibiotics (100 U/mL penicillin and 100 mg/mL streptomycin) and cultured in an incubator with 5% CO2 at 37 °C.

A psoriasis-like keratinocytes model was established by adding M5 cocktail cytokines (known to be involved in the genesis of the psoriatic phenotype (IL-17A, IL-22, oncostatin M, IL-1α, and TNF-α, each at a final concentration of 2.5 ng/mL) into the medium of HaCaT keratinocytes. This mixture has been shown to elicit many features of the psoriatic phenotype. This mixture was incubated for 72 hours as a control.

HaCaT cells were seeded at approximately 50% density (1x104 cells/well) and after 16 h the medium was replaced with 50 µL of pre-treatment medium containing different concentrations of allopurinol (0, 0.5, or 3 mM) or methotrexate (1 µM) and placed back into the incubator. Treatment media was prepared using standard media containing vehicle control (PBS) or M5 cocktail.

Other HaCaT cells were incubated in vitro for 72 hours in the presence of a M5 composition that also included 1.0 µM methotrexate, 0.5 mM allopurinol, 3.0 mM allopurinol, 0.5 mM oxypurinol, or 3.0 mM oxypurinol. The allopurinol, oxypurinol, and methotrexate were all obtained from Sigma (St. Louis, MO). Each was taken up in a 1N NaOH solution and then diluted to the appropriate concentrations in cell culture media.

The number of live and dead cells were determined by the MultiTox-Glo Multiplex Cytotoxicity Assay (Promega, Madison WI) according to the manufacturer's protocol. A dilution series of cells was used to generate a standard curve for calculation of cell number in each well. The total number of cells was the sum of the live and dead cells.

Table 1 shows the live and dead cell counts of the HaCaT cells at 0 hours and total and dead cell counts at 48 hours and at 72 hours, with standard deviations listed as well. The total cell count is the sum of the live cell count and the dead cell count. Methotrexate was the comparator therapeutic for topical therapy of psoriasis at the standard 1 µM dose. The ideal result of the cell culture would be to have the total cell number neither increase nor decrease over time. An increase in total cell number indicates that the psoriatic phenotype is not suppressed. A decrease in total cell number indicates cell toxicity from the treatment.

Regarding dead cells, the ideal result would be to have as close to zero dead cells as possible. An increase in the number of dead cells indicates cell toxicity. Note that the lowest observed number of total cells was for 3.0 mM allopurinol. Note that at 72 hours, 3.0 mM allopurinol has both the lowest number total cells and the lowest number of dead cells.

**Table 1: Cell Counts**

| | 0 hours | | 48 hours | | 72 hours | |
|---|---|---|---|---|---|---|
| | Live | Dead | Total | Dead | Total | Dead |
| Control | 9177±90 | 801±19 | 15426±123 | 3034±43 | 17298±444 | 5099±384 |
| 0.5 mM oxypurinol | 9177±90 | 801±19 | 14960±100 | 2937±65 | 17036±222 | 4761±200 |
| 3.0 mM oxypurinol | 9177±90 | 801±19 | 13069±142 | 2508±124 | 14228±282 | 3691±244 |
| 0.5 mM allopurinol | 9177±90 | 801±19 | 11705±161 | 2319±141 | 13428±216 | 4024±166 |
| 3.0 mM allopurinol | 9177±90 | 801±19 | 9038±176 | 1275±117 | 9367±280 | 2004±168 |
| 1.0 µM methotrexate | 9177±90 | 801±19 | 11194±545 | 4826±464 | 11587±593 | 6300±289 |

The raw data of Table 1 were converted to the relative data of the cell ratios of Table 2. The cell ratios in Table 2 were obtained by dividing the total and dead cell numbers in Table 1 by 9177, the number of live cells at zero hours. The ideal result for the total cell ratio would be to have the total cell ratio to be about 1 and neither increase nor decrease over time. The ideal result for the dead cell ratio would be to have the dead cell ratio as close to zero as possible with no increase over time.

The total cell ratio values of Table 2 are plotted in FIG. 1. For the control M5-treated cells 10, the total cell ratio is 1.89 or an 89% increase over the initial number of live cells during the 72-hour incubation period. For 0.5 mM oxypurinol 20, the total cell ratio at 72 hours is similarly 1.86 of the initial number of live cells. For methotrexate 40, the total cell ratio at 72 hours is 1.26 of the initial number of live cells. For 3.0 mM oxypurinol 25, the total number of cells at 72 hours is 1.55 of the initial number of live cells. For 0.5 mM allopurinol 30, the total number of cells at 72 hours is 1.46 of the initial number of live cells. For 3.0 mM allopurinol 35, the total number of cells at 72 hours is only 1.02 of the initial number of live cells or a very slight increase.

**Table 2: Cell Ratios**

| | 0 hours | | 48 hours | | 72 hours | |
|---|---|---|---|---|---|---|
| | Total | Dead | Total | Dead | Total | Dead |
| Control | 1.087 | 0.087 | 1.681 | 0.331 | 1.885 | 0.556 |
| 0.5 mM oxypurinol | 1.087 | 0.087 | 1.630 | 0.320 | 1.856 | 0.519 |
| 3.0 mM oxypurinol | 1.087 | 0.087 | 1.424 | 0.273 | 1.550 | 0.402 |
| 0.5 mM allopurinol | 1.087 | 0.087 | 1.275 | 0.253 | 1.463 | 0.438 |
| 3.0 mM allopurinol | 1.087 | 0.087 | 0.985 | 0.139 | 1.021 | 0.218 |
| 1.0 µM methotrexate | 1.087 | 0.087 | 1.220 | 0.526 | 1.263 | 0.686 |

The dead cell ratio values of Table 2 are plotted in FIG. 2. The control value 50 for the dead cell ratio after 72 hours is 0.56 or 56% of the input number of live cells. Methotrexate 80 results in a higher ratio of 0.69 or 69% of the input live cells being dead. Surprisingly, the percentage of dead cells was lower for allopurinol than for the control, oxypurinol, and methotrexate. The percentage was even lower when increasing the concentration of allopurinol from 0.5 mM to 3.0 mM. For 0.5 mM allopurinol 70, the dead cell ratio is 0.44, and for 3 mM allopurinol 75, the dead cell ratio is only 0.22. Again, completely unexpected is the reduced benefit of the active species oxypurinol, with a dead cell ratio of 0.52 for 0.5 mM oxypurinol 60 and 0.40 for 3.0 mM oxypurinol 65. Importantly, and in contrast to methotrexate, allopurinol at 3.0 mM decreases cell proliferation without increasing cell death.

In summary, the results in FIG. 1 and FIG. 2 for this widely used model of psoriasis show that allopurinol can control cell proliferation much more effectively than both its metabolite oxypurinol and a standard agent in clinical use, methotrexate. Allopurinol therefore represents an option for therapy that can halt the psoriatic process.

### Example 2

The growth effects of allopurinol and/or glutathione, with Vitamin D being included as a control, were examined on a MatTek human PSE culture system (SOR-300-FT) that included a stratum corneum layer, an epidermis layer, and a dermis layer. The growth conditions of the psoriatic tissue were according to the proprietary conditions of the manufacturer, MatTek. The system included a dermal layer of psoriatic origin covered by a normal epidermal cell layer. The stratum corneum is an acellular superficial keratin layer. The cellular epidermis underlay the stratum corneum and covered the deep dermis layer. Only the dermis layer in this system was of psoriatic origin. Psoriasis was marked by an increase in thickness of all three layers.

Six MatTek human PSE culture systems were exposed for five days to four different treatment compositions. The first treatment composition was a negative control. The second treatment composition included 0.5 mM allopurinol. The third treatment composition included 3.0 mM allopurinol. The fourth treatment composition included 3.0 mM glutathione. The fifth treatment composition included a combination of 3.0 mM allopurinol and 3.0 mM glutathione. The sixth treatment composition included 100 nM Vitamin D as a control. The allopurinol, glutathione, and Vitamin D were all obtained from Sigma (St. Louis, MO). Each was taken up in a 1N NaOH solution and then diluted to the appropriate concentrations in cell culture media.

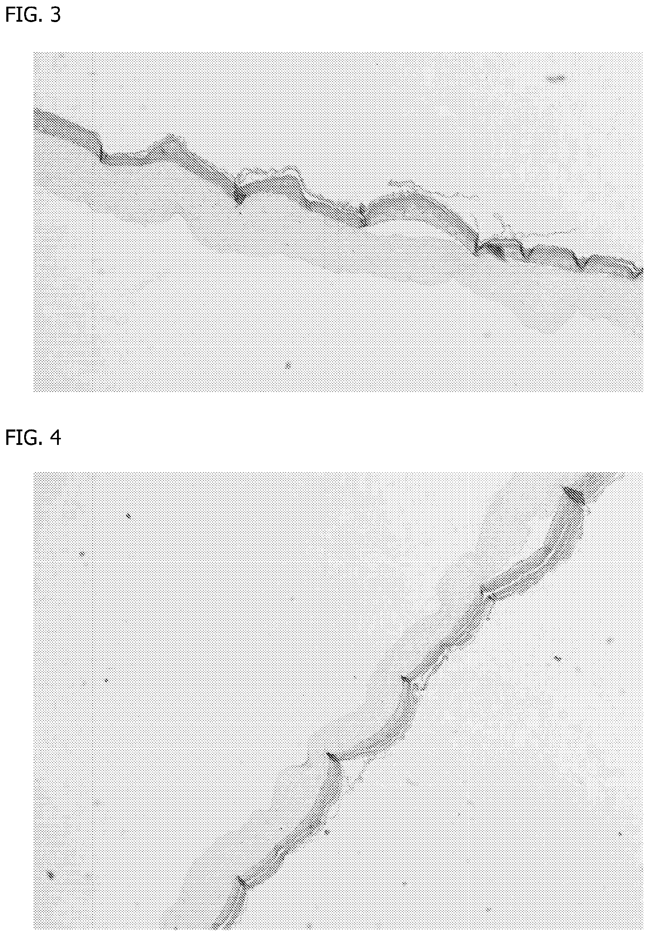

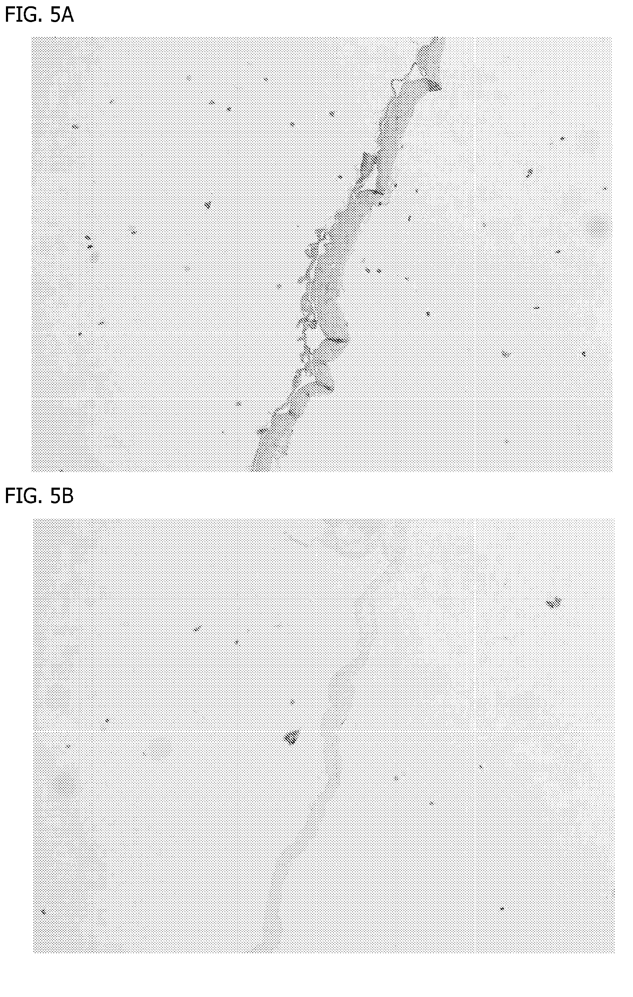

FIG. 3 shows a MatTek human PSE culture system after five days of exposure to the third treatment composition. FIG. 4 shows a MatTek human PSE culture system after five days of exposure to the fifth treatment composition. FIG. 5A and FIG. 5B show a MatTek human PSE culture system after five days of exposure to the sixth treatment composition. The images of FIG. 3, FIG. 4, FIG. 5A, and FIG. 5B are at the same magnification. The dermal layer is the lighter layer and the epidermal layer is the darker layer. The photographs show the decrease in the thickness of the dermal layer in the presence of both glutathione and allopurinol (FIG. 4) compared with allopurinol alone (FIG. 3). FIG. 5A and FIG. 5B show the separation of dermal and epidermal layers resulting from Vitamin D treatment and may reflect the known toxicity of Vitamin D therapy.

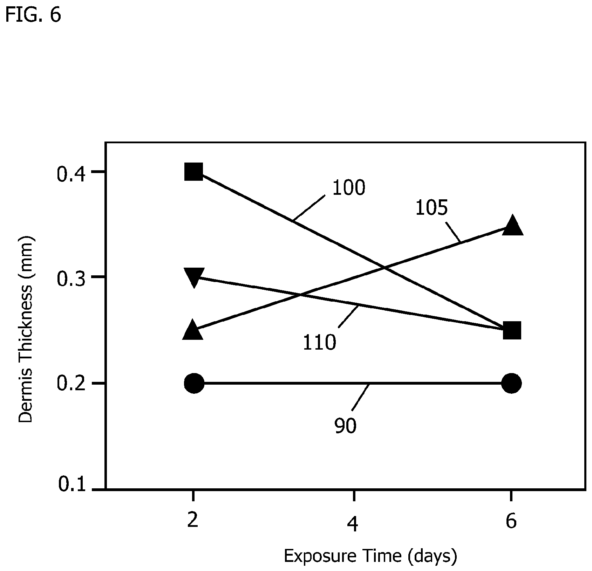

The thickness of each of the three tissue layers of skin was monitored and measured after two days and after six days of exposure to the treatment medium in a blinded fashion by a board-certified and nationally respected pathologist. The resulting measurements are shown in Table 3 and FIG. 6.

An increase in the thickness of the dermis is a feature of psoriasis (see, for example, Phillips et al., "Dermal Reflectivity Determined by Optical Coherence Tomography is an Indicator of Epidermal Hyperplasia and Dermal Edema within Inflamed Skin", Journal of Biomedical Optics, Vol. 16, Art. 040503 (2011)) and an unexpected result of glutathione treatment in Table 3. Table 3 shows that treatment with a combination of allopurinol and glutathione actually decreases the thickness of the dermis from two days to six days. It is interesting that a benefit of the allopurinol and glutathione involved a specific reversal in the psoriatic dermal layer thickening. Also, tissue integrity with the treatment with both allopurinol and glutathione was preserved. The treatment with the active agent Vitamin D led to the separation of a thinned dermis and the epidermis layers, possibly evidence of toxicity that is a known side effect of Vitamin D therapy.

**Table 3: Skin Tissue Thickness**

| Layer | Time | Layer Thickness (mm) | | | | | |
|---|---|---|---|---|---|---|---|
| Treatment | | Control | 0.5 mM allopurinol | 3 mM allopurinol | 3 mM glutathione | 3 mM allopurinol + 3mM glutathione | 100 nM Vitamin D |
| Stratum Corneum | 2 days | 0.02 | 0.05 | 0.02 | 0.15 | 0.05 | 0.05 |
| | 6 days | 0.15 | 0.05 | 0.1 | N/D | 0.1 | 0.2 |
| Epidermis | 2 days | 0.1 | 0.1 | 0.1 | 0.2 | 0.1 | 0.15 |
| | 6 days | 0.2 | 0.1 | 0.15 | N/D | 0.15 | 0.2 |
| Dermis | 2 days | 0.2 | 0.4 | 0.25 | 0.3 | 0.3 | 0.25 |
| | 6 days | 0.2 | 0.25 | 0.35 | N/D | 0.25 | 0.2 |

FIG. 6 shows that 3.0 mM of allopurinol alone 100 caused a 1.4 fold increase in dermis layer thickness during this time interval from treatment for 2 days to 6 days. In contrast, the treatment with a combination of 3.0 mM allopurinol and 3.0 mM glutathione 110 decreased the thickness of the dermis to 0.83 during the same time interval. The control 90 showed no dermis layer thickening. As is seen in FIG. 6, increasing from 0.5 mM allopurinol 105 to 3 mM allopurinol 110 significantly increased day 6 dermis thickening. Thus, there is a specific effect of the combination of allopurinol and glutathione that reversed the psoriatic dermis layer thickening seen with 3.0 mM of allopurinol treatment alone.

Additionally, the tissue integrity with the treatment with both allopurinol and glutathione was preserved. Combined treatment with allopurinol and glutathione at 3mM resulted in no keratinocyte toxicity (dyskeratosis) and decreased cell presence in the keratin layer (parakeratosis) after 6 days compared with allopurinol treatment alone.

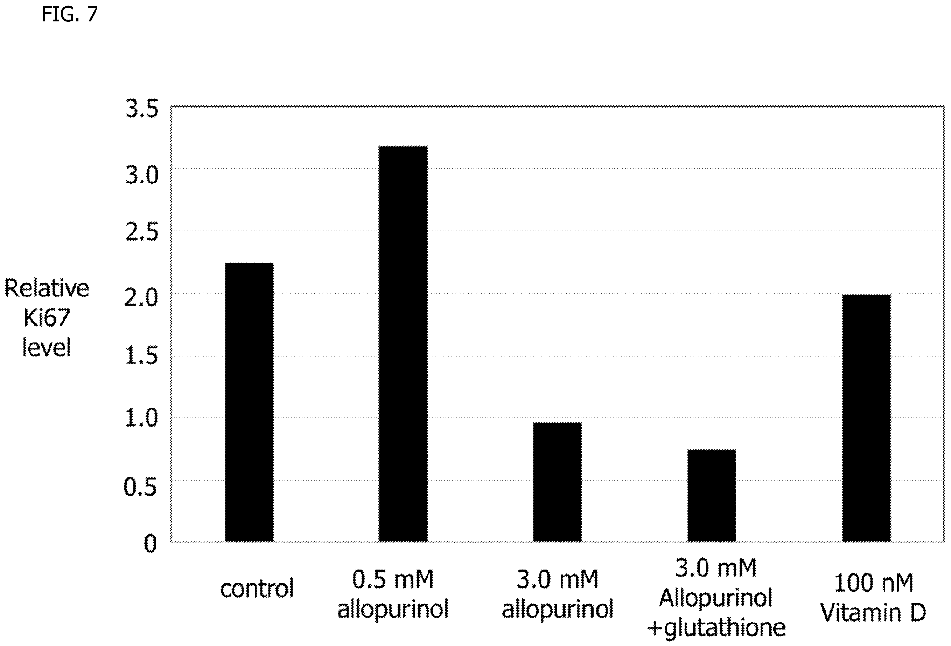

The level of Ki67 in the MatTek tissue culture system was also determined. Ki67 is a nuclear protein and a replication marker. The level of Ki67 was determined by polymerase chain reaction (PCR) analysis with standard protocols. The measured relative Ki67 levels at Day 6 are shown in Table 4 and FIG. 7. The relative levels are normalized with respect to the measured Ki67 level of the control at Day 0, which is adjusted to 1.0.

The Ki67 level is an indicator of cell replication rate, with a lower value indicating a lower replication rate. Table 4 shows that treatment with 3 mM allopurinol and treatment with a combination of 3 mM allopurinol and 3 mM glutathione reduced the Ki67 level relative to the control, with the combination performing slightly better than 3 mM allopurinol alone and much better than 100 nM Vitamin D.

**Table 4: Relative Replication Levels**

| Treatment | Relative Ki67 Level |
|---|---|
| Control | 2.24 |
| 0.5 mM allopurinol | 3.18 |
| 3 mM allopurinol | 0.96 |
| 3 mM allopurinol + 3mM glutathione | 0.74 |
| 100 nM Vitamin D | 1.99 |

Other markers of cell proliferation were also measured, namely (protein p21), and four markers of inflammatory signaling (IL17A, IL23A, TNF-α, and transforming growth factor beta (TGF-β)) in the MatTek PSE culture system after two days of treatment were also determined. Ki67 is a nuclear protein and a replication marker. The marker levels were determined by isolating total RNA by a Promega SV96 Total RNA Isolation Vacuum System (Promega, Madison, WI) according to the manufacturer's instructions. The total RNA was reverse transcribed using an ABI High Capacity cDNA reverse transcription kit (Applied Biosystems, Foster City, CA). Quantitative real-time polymerase chain reaction (qPCR) was performed with the use of a SYBR Green PCR Master Mix (Applied Biosystems) according to the manufacturer's protocol and amplified on a StepOnePlus Real-Time PCR System.

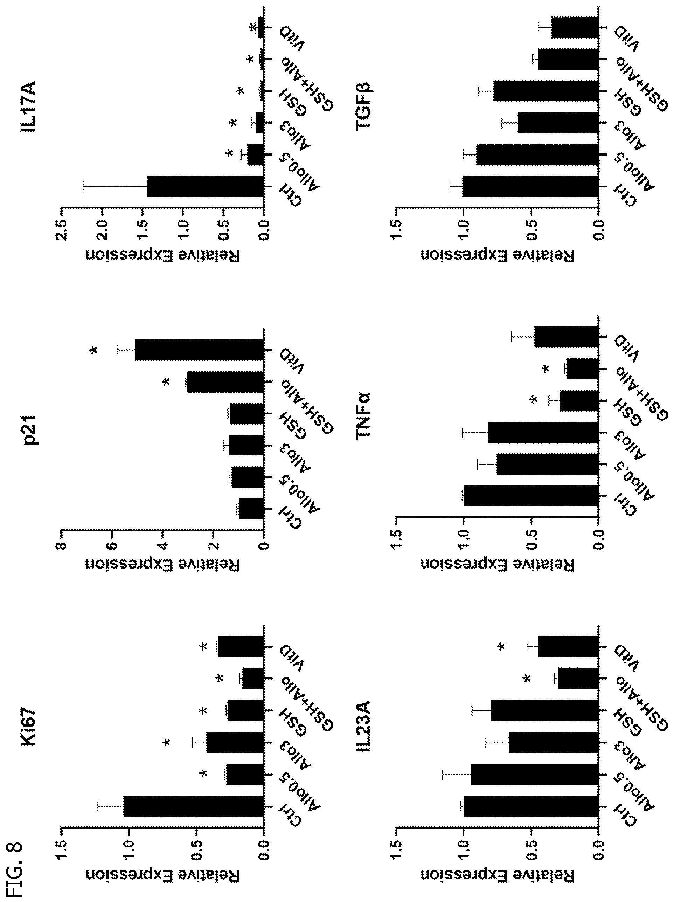

The measured relative marker levels after two days of treatment are shown in Table 5 and FIG. 8.

**Table 5: Relative Expression Levels**

| Treatment | Ki67 | p21 | IL17A | IL23A | TNF-α | TGF-β |
|---|---|---|---|---|---|---|
| Control | 1.03±0.19 | 1.00±0.06 | 1.44±0.79 | 1.00±0.02 | 1.00±0.01 | 1.00±0.08 |
| 0.5 mM allopurinol | 0.27±0.01 | 1.27±0.10 | 0.21±0.07 | 0.95±0.20 | 0.75±0.13 | 0.90±0.09 |
| 3 mM allopurinol | 0.42±0.09 | 1.40±0.17 | 0.09±0.04 | 0.66±0.17 | 0.82±0.19 | 0.60±0.12 |
| 3 mM glutathione | 0.27±0.01 | 1.35±0.06 | 0.04±0.02 | 0.79±0.14 | 0.29±0.07 | 0.77±0.10 |
| 3 mM allopurinol + 3 mM glutathione | 0.16±0.02 | 3.06±0.03 | 0.03±0.01 | 0.29±0.03 | 0.23±0.01 | 0.45±0.04 |
| 100 nM Vitamin D | 0.34±0.01 | 5.10±0.70 | 0.06±0.03 | 0.45±0.07 | 0.48±0.17 | 0.35±0.09 |

Unexpectedly, as shown in Table 5 and FIG. 8, after 2 days of treatment, the PSE cells with allopurinol or glutathione individually significantly decreased Ki67, IL17A, IL23A, and TNFα mRNA levels while increasing the expression of cell replication inhibitory gene p21. To highlight effects on cell proliferation, the ratio of p21 to Ki67 mRNA levels were calculated, with the results being shown in FIG. 9. After two days of treatment, both the allopurinol and glutathione alone and the synergistic combination of allopurinol and glutathione, as well as Vitamin D in parallel treatment, demonstrated a markedly increased p21/Ki67 ratio, indicative of decreased cell replication.

### Example 3

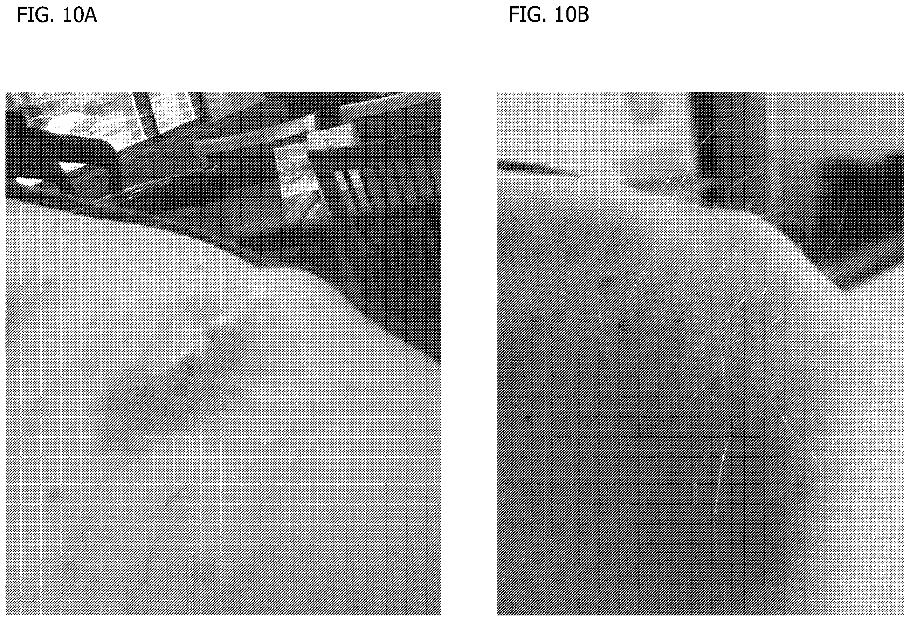

A psoriatic plaque on the left shoulder had persisted for more than two years. FIG. 10A shows the plaque prior to treatment.

A treatment composition of about 200 µL of 3% allopurinol and 3% glutathione suspended in a petrolatum-based ointment was applied twice daily to the psoriatic plaque for one month. Both the allopurinol and the glutathione were United States Pharmacopeia (USP) grade. The allopurinol was in the form of ground pills mixed to the proper concentration in the petrolatum-based ointment. The glutathione was in the form of a powder first mixed sparingly with mineral oil and then to the proper concentration in the petrolatum-based ointment. The petrolatum-based ointment also included water, mineral oil, ceresin, lanolin alcohol, panthenol, glycerin, and bisabolol as inactive ingredients. FIG. 10B shows that the left shoulder completely responded to the treatment, with the plaque no longer being visible. No side effects of the treatment were observed or reported.

### Example 4

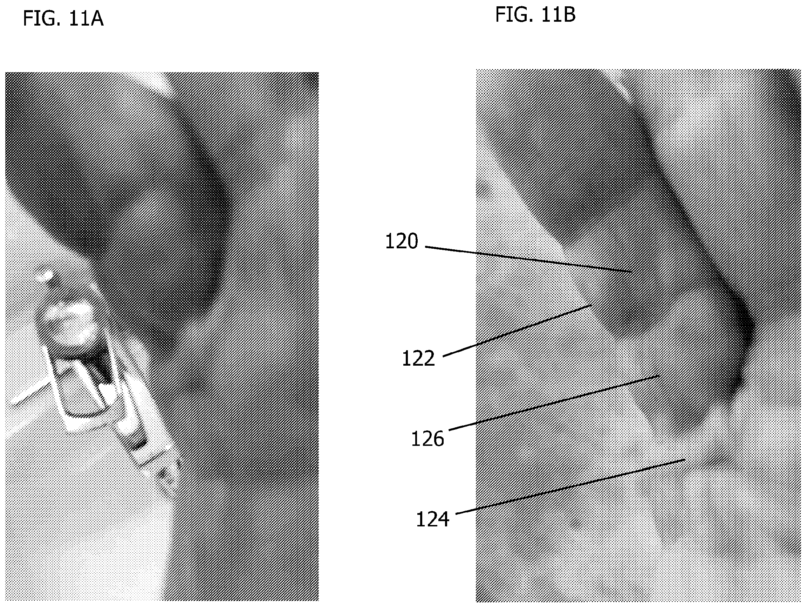

A Dupuytren's contracture is an abnormal skin thickening, typically of the palm at the base of the fingers. The thickening may deform a finger to curl or turn toward the side or toward the palm. FIG. 11A shows a Dupuytren's contracture of the right fifth finger of the Applicant prior to treatment. FIG. 11B shows the middle phalanx 120 of the little finger having thickened overlying skin 122 as well as thickened skin overlying the tissue of the distal head of the fifth metacarpal 124 and thickened skin over the proximal phalanx 126 characteristic of a Dupuytren's contracture.

A treatment composition of about 200 µL of 3% allopurinol suspended in a petrolatum-based ointment was applied twice daily to the thickened skin for one month. The allopurinol was USP grade. The allopurinol was in the form of ground pills mixed to the proper concentration in the petrolatum-based ointment. FIG. 11B shows that the finger responded with somewhat improved movement.

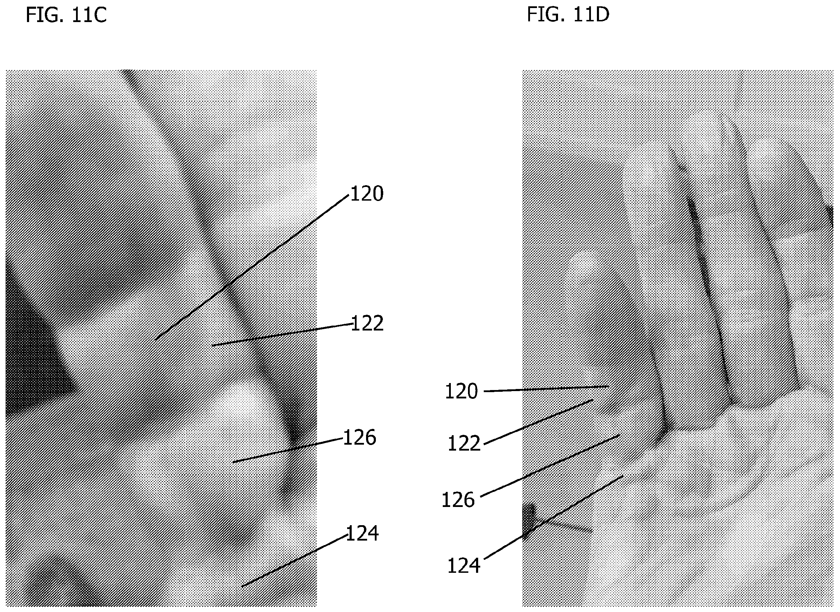

Subsequently, a treatment composition of about 200 µL of 3% allopurinol and 3% glutathione suspended in a petrolatum-based ointment was applied twice daily to the psoriatic plaque for an additional month. The glutathione was USP grade. The glutathione was in the form of a powder first mixed sparingly with mineral oil and then to the proper concentration in the petrolatum-based ointment. FIG. 11C shows that after the second month, the right fifth finger was functionally near normal with decreased swelling. The right fifth finger was fully mobile but remained foreshortened by about 1 cm compared with the left fifth finger after more than 2 months of treatment. No side effects of the treatment were observed or reported.

The progressive changes over four months of continuous human use of daily topical 10% allopurinol and 10% glutathione suspended in a petrolatum-based ointment in the treatment of Dupuytren's contracture are manifest in the thickness of the tissues that decreases gradually but progressively over time. FIG. 11D shows that the middle phalanx 120 of the little finger shows continued shrinkage of the overlying skin 122. The skin overlying the tissue of the distal head of the fifth metacarpal 124 also shows skin shrinkage as well as the skin over the proximal phalanx 126. There was gradual progressive increase in mobility of the right fifth finger with strength at least equivalent to that of the left (dominant side) fifth finger. No side effects were noted.

### Example 5

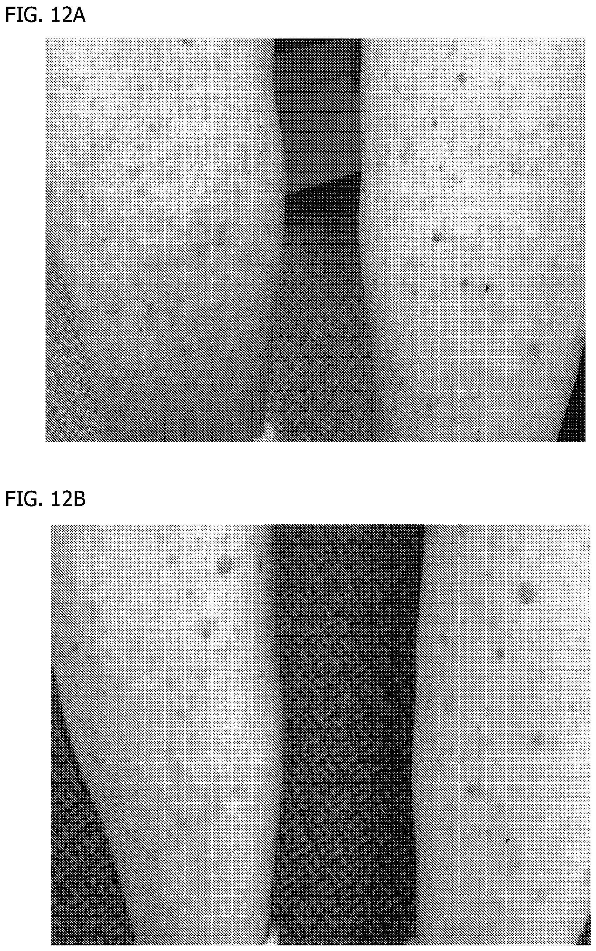

FIG. 12A shows a widespread rash on the backs of the calves of the legs that started about two months earlier as an allergic reaction.

A treatment composition of 3% allopurinol suspended in a petrolatum-based ointment was applied twice daily to the left calf and a treatment composition of 3% allopurinol and 3% glutathione suspended in a petrolatum-based ointment was applied twice daily to the right calf. Both the allopurinol and the glutathione were USP grade. The allopurinol was in the form of ground pills mixed to the proper concentration in the petrolatum-based ointment. The glutathione was in the form of a powder mixed sparingly with mineral oil and then to the proper concentration in the petrolatum-based ointment.

FIG. 12B shows the calves after five days of treatment. The right calf, treated with the combination of 3% allopurinol and 3% glutathione, shows more improvement than the left calf, treated with only 3% allopurinol.

The recurrent rash responded rapidly to a further treatment with 3% allopurinol and 3% glutathione applied twice daily. The itching resolved within 24 hours of application of the further treatment, and the swelling began to diminish within 48 hours, with continued response thereafter.

### Example 6

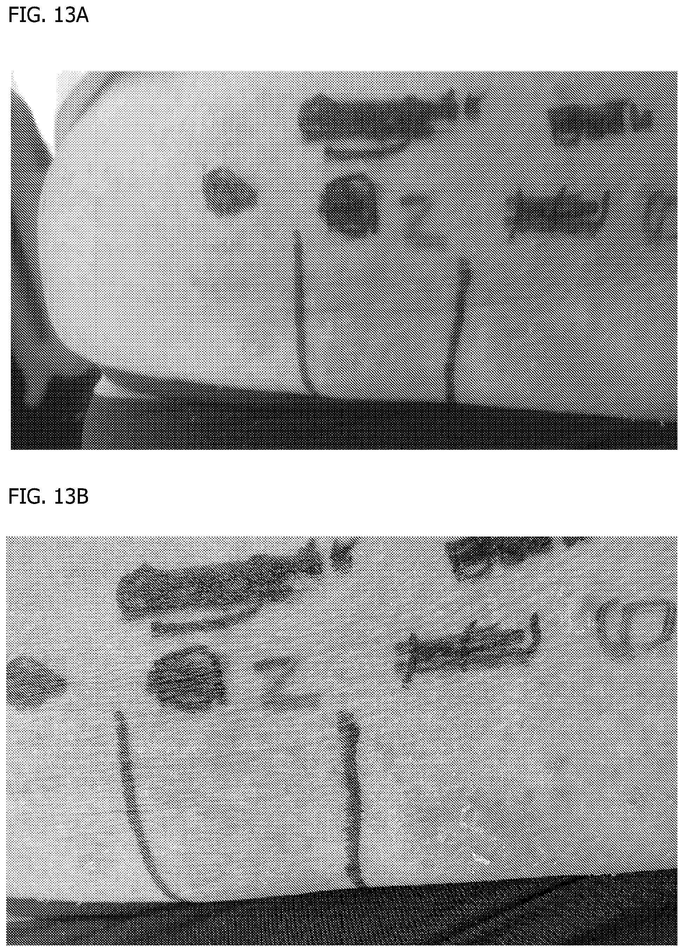

FIG. 13A shows three areas of guttate psoriasis, a more acute form of psoriasis than plaque psoriasis, prior to treatment.

No treatment was applied to the leftmost area of guttate psoriasis. A treatment composition of 3% allopurinol suspended in a petrolatum-based ointment was applied twice daily to the center area of guttate psoriasis. A treatment composition of 3% allopurinol and 3% glutathione suspended in a petrolatum-based ointment was applied twice daily to the rightmost area of guttate psoriasis. Both the allopurinol and the glutathione were USP grade. The allopurinol was in the form of ground pills mixed to the proper concentration in the petrolatum-based ointment. The glutathione was in the form of a powder mixed sparingly with mineral oil and then to the proper concentration in the petrolatum-based ointment.

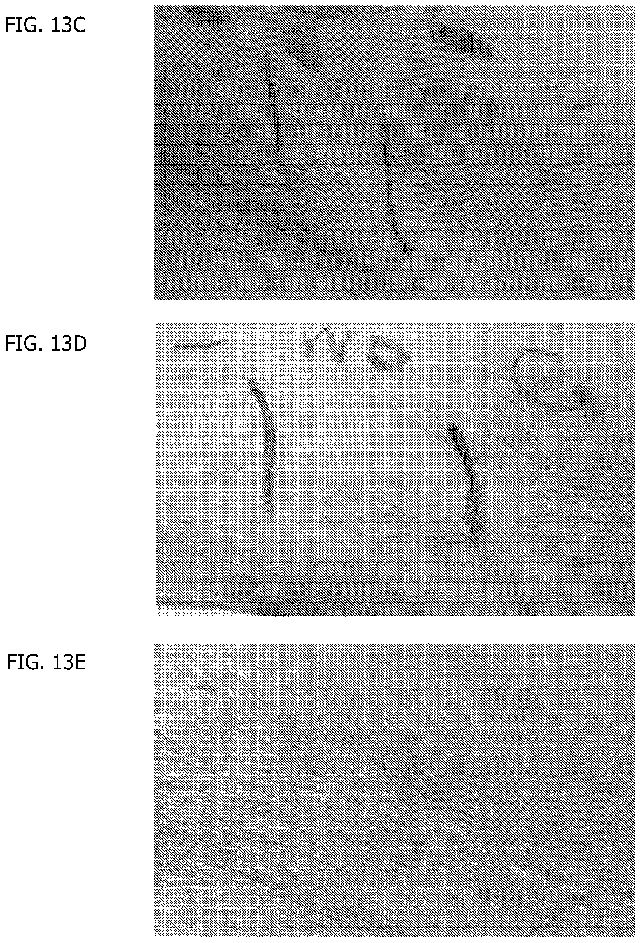

FIG. 13B, FIG. 13C, FIG. 13D, and FIG. 13E show the three areas after one and a half days, two days, two and a half days, and six and a half days after the start of treatment.

FIG. 13C shows that after two days of treatment, the lesion in the center area treated with both allopurinol and glutathione was much improved and nearly flat.

### Example 7

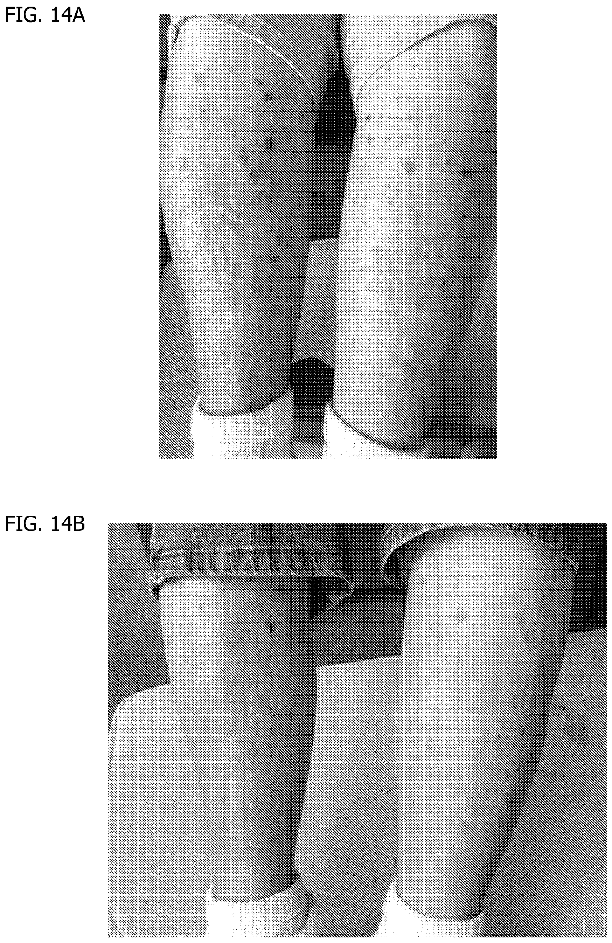

FIG. 14A shows an allergic reaction with fluid weeping from both legs prior to treatment.

For seven days, a treatment composition of 3% allopurinol suspended in a petrolatum-based ointment was applied twice daily to the individual's left leg, and a treatment composition of 3% allopurinol and 3% glutathione suspended in a petrolatum-based ointment was applied twice daily to the individual's right leg. Both the allopurinol and the glutathione were USP grade. The allopurinol was in the form of ground pills mixed to the proper concentration in the petrolatum-based ointment. The glutathione was in the form of a powder mixed sparingly with mineral oil and then to the proper concentration in the petrolatum-based ointment.

FIG. 14B shows that after seven days of treatment, the treated skin on both legs showed significant improvement. Although not quantitatively measured, the treated right leg that received the treatment composition including both allopurinol and glutathione showed a dramatic improvement in the definition of the right tibial bone compared with prior to treatment.

All references mentioned herein are hereby incorporated by reference herein.

While the invention has been described with reference to one or more embodiments, it will be understood by those skilled in the art that various changes may be made and equivalents may be substituted for elements thereof without departing from the scope of the claims. In addition, many modifications may be made to adapt a particular situation or material to the teachings of the invention without departing from the essential scope of the claims. Therefore, it is intended that the invention not be limited to the particular embodiment disclosed as the best mode contemplated for carrying out this invention, but that the invention will include all embodiments falling within the scope of the appended claims. In addition, all numerical values identified in the detailed description shall be interpreted as though the precise and approximate values are both expressly identified.

## Claims

1. A topical pharmaceutical formulation comprising allopurinol or a pharmaceutically acceptable salt thereof and a co-agent or a pharmaceutically acceptable salt thereof, wherein the co-agent is glutathione, the topical pharmaceutical formulation further comprising a carrier.

2. The topical pharmaceutical formulation of claim 1, wherein the amount of allopurinol or a pharmaceutically acceptable salt thereof is between 1% and 10% by weight.

3. The topical pharmaceutical formulation of claim 1 or 2, wherein the amount of glutathione or a pharmaceutically acceptable salt thereof is between 1% and 10% by weight.

4. The topical pharmaceutical formulation of any of claims 1-3, wherein the pharmaceutical formulation is anhydrous, aqueous, or an emulsion.

5. The topical pharmaceutical formulation of any of claims 1-4, wherein the only active ingredients are allopurinol and glutathione or pharmaceutically acceptable salts thereof.

6. The topical pharmaceutical formulation of any of claims 1-5, further comprising one or more additional active ingredients.

7. The topical pharmaceutical formulation of any one of claim 1-6, further comprising one or more pharmaceutically acceptable excipients.

8. The topical pharmaceutical formulation of any of claim 1-7 for use in treating a skin disorder.

9. The topical pharmaceutical formulation of claim 8, wherein the skin disorder is a proliferative skin disorder.

10. The topical pharmaceutical formulation of claim 9, wherein the proliferative skin disorder is psoriasis.

11. The topical pharmaceutical formulation of claim 9, wherein the proliferative skin disorder is a cutaneous cellular proliferative disorder.

12. The topical pharmaceutical formulation of claim 9, wherein the proliferative skin disorder is selected from the group consisting of cutaneous psoriasis, keratoacanthoma, rosacea, keloids, hand-foot syndrome, and cancer.

13. The topical pharmaceutical formulation of claim 9, wherein the skin disorder is Dupuytren's contracture.

14. The topical pharmaceutical formulation of claim 9, wherein the skin disorder is an allergic reaction.

15. The topical pharmaceutical formulation of claim 9, wherein the allergic reaction is a cutaneous allergic reaction.

## Patentansprüche

1. Topische pharmazeutische Formulierung, die Allopurinol oder ein pharmazeutisch verträgliches Salz davon und ein Co-Agens oder ein pharmazeutisch verträgliches Salz davon umfasst, wobei es sich bei dem Co-Agens um Glutathion handelt und die topische pharmazeutische Formulierung ferner einen Trägerstoff umfasst.

2. Topische pharmazeutische Formulierung nach Anspruch 1, wobei die Menge an Allopurinol oder an einem pharmazeutisch verträglichen Salz davon zwischen 1 und 10 Gew.-% liegt.

3. Topische pharmazeutische Formulierung nach Anspruch 1 oder 2, wobei die Menge an Glutathion oder an einem pharmazeutisch verträglichen Salz davon zwischen 1 und 10 Gew.-% liegt.

4. Topische pharmazeutische Formulierung nach einem der Ansprüche 1 bis 3, wobei die pharmazeutische Formulierung wasserfrei, wässrig oder eine Emulsion ist.

5. Topische pharmazeutische Formulierung nach einem der Ansprüche 1 bis 4, wobei die einzigen Wirkstoffe Allopurinol und Glutathion oder pharmazeutisch verträgliche Salze davon sind.

6. Topische pharmazeutische Formulierung nach einem der Ansprüche 1 bis 5, die ferner einen oder mehrere zusätzliche Wirkstoffe umfasst.

7. Topische pharmazeutische Formulierung nach einem der Ansprüche 1 bis 6, die ferner einen oder mehrere pharmazeutisch verträgliche Hilfsstoffe umfasst.

8. Topische pharmazeutische Formulierung nach einem der Ansprüche 1 bis 7 zur Verwendung bei der Behandlung einer Hauterkrankung.

9. Topische pharmazeutische Formulierung nach Anspruch 8, wobei es sich bei der Hauterkrankung um eine proliferative Hauterkrankung handelt.

10. Topische pharmazeutische Formulierung nach Anspruch 9, wobei es sich bei der proliferativen Hauterkrankung um Psoriasis handelt.

11. Topische pharmazeutische Formulierung nach Anspruch 9, wobei es sich bei der proliferativen Hauterkrankung um eine kutane zelluläre proliferative Erkrankung handelt.

12. Topische pharmazeutische Formulierung nach Anspruch 9, wobei die proliferative Hauterkrankung aus der aus kutaner Psoriasis, Keratoakanthom, Rosazea, Keloiden, Hand-Fuß-Syndrom und Krebs bestehenden Gruppe ausgewählt ist.

13. Topische pharmazeutische Formulierung nach Anspruch 9, wobei es sich bei der Hauterkrankung um die Dupuytren-Kontraktur handelt.

14. Topische pharmazeutische Formulierung nach Anspruch 9, wobei es sich bei der Hauterkrankung um eine allergische Reaktion handelt.

15. Topische pharmazeutische Formulierung nach Anspruch 9, wobei es sich bei der allergischen Reaktion eine kutane allergische Reaktion handelt.

## Revendications

1. Formulation pharmaceutique topique comprenant de l'allopurinol ou un sel pharmaceutiquement acceptable de celui-ci et un co-agent ou un sel pharmaceutiquement acceptable de celui-ci, le co-agent étant le glutathion, la formulation pharmaceutique topique comprenant en outre un agent porteur.

2. Formulation pharmaceutique topique selon la revendication 1, la quantité d'allopurinol ou d'un sel pharmaceutiquement acceptable de celui-ci étant comprise entre 1 % et 10 % en poids.

3. Formulation pharmaceutique topique selon la revendication 1 ou 2, la quantité de glutathion ou d'un sel pharmaceutiquement acceptable de celui-ci étant comprise entre 1 % et 10 % en poids.

4. Formulation pharmaceutique topique selon l'une des revendications 1 à 3, la formulation pharmaceutique étant anhydre, aqueuse ou une d'émulsion.

5. Formulation pharmaceutique topique selon l'une des revendications 1 à 4, les seuls principes actifs étant l'allopurinol et le glutathion ou leurs sels pharmaceutiquement acceptables.

6. Formulation pharmaceutique topique selon l'une des revendications 1 à 5, comprenant en outre un ou plusieurs principes actifs supplémentaires.

7. Formulation pharmaceutique topique selon l'une des revendications 1 à 6, comprenant en outre un ou plusieurs excipients pharmaceutiquement acceptables.

8. Formulation pharmaceutique topique selon l'une des revendications 1 à 7 pour l'utilisation dans le traitement d'un trouble cutané.

9. Formulation pharmaceutique topique selon la revendication 8, le trouble cutané étant un trouble cutané prolifératif.

10. Formulation pharmaceutique topique selon la revendication 9, le trouble cutané prolifératif étant le psoriasis.

11. Formulation pharmaceutique topique selon la revendication 9, le trouble cutané prolifératif étant une maladie cutanée à prolifération cellulaire.

12. Formulation pharmaceutique topique selon la revendication 9, le trouble cutané prolifératif étant choisi dans le groupe constitué du psoriasis cutané, du kératoacanthome, de la rosacée, des chéloïdes, du syndrome main-pied et du cancer.

13. Formulation pharmaceutique topique selon la revendication 9, le trouble cutané étant la contracture de Dupuytren.

14. Formulation pharmaceutique topique selon la revendication 9, le trouble cutané étant une réaction allergique.

15. Formulation pharmaceutique topique selon la revendication 9, la réaction allergique étant une réaction allergique cutanée.
